# EUROPEAN PATENT SPECIFICATION

(11) **EP 1 941 026 B1**
(45) Date of publication and mention of the grant of the patent: **04.06.2014**
(21) Application number: 06844211.0
(22) Date of filing: 24.10.2006
(51) Int. Cl.: C12N 5/071

(54) **METHODS OF PROTEIN PRODUCTION USING ANTI-SENESCENCE COMPOUNDS**
VERFAHREN ZUR PROTEINHERSTELLUNG MIT ANTI-SENESZENZVERBINDUNGEN
PROCEDES DE PRODUCTION DE PROTEINES UTILISANT DES COMPOSES ANTI-SENESCENCE

(30) Priority: 24.10.2005 US 729573 P
(43) Date of publication of application: 09.07.2008
(73) Proprietor: Wyeth LLC, New York, NY 10017-5755 (US)
(72) Inventor: LUAN, Yen-Tung, Chelmsford, MA 01824 (US); WANG, Wenge, North Chelmsford, MA 01863 (US); THODAY, Paul, Sterling, MA 01564 (US); DRAPEAU, Denis, Salem, NH 03079 (US); CHOU, Judy, Lexington, MA 02421 (US)
(74) Representative: Pfizer
(86) International application number: PCT/US2006/041256
(87) International publication number: WO 2007/050498

(56) References cited:
- WO-A-92/09298
- WO-A-2005/064013
- CALABRESE V ET AL: "Protective Effect of Carnosine During Nitrosative Stress in Astroglial Cell Cultures" NEUROCHEMICAL RESEARCH, KLUWER ACADEMIC PUBLISHERS-PLENUM PUBLISHERS, NE, vol. 30, no. 6-7, 1 June 2005 (2005-06-01), pages 797-807, XP019289871 ISSN: 1573-6903
- KARL BAUER: "Carnosine and Homocarnosine, the Forgotten, Enigmatic Peptides of the Brain" NEUROCHEMICAL RESEARCH, KLUWER ACADEMIC PUBLISHERS-PLENUM PUBLISHERS, NE, vol. 30, no. 10, 1 October 2005 (2005-10-01), pages 1339-1345, XP019289763 ISSN: 1573-6903

## Description

### BACKGROUND OF INVENTION

This disclosure relates generally to the production of proteins in mammalian cell cultures. Particularly this disclosure relates to culturing mammalian cells in the presence of an anti-senescence compound, e.g. carnosine, to maintain viability and increase productivity with superior quality. Culturing cells has produced many protein products. These products, such as hybridoma-produced monoclonal antibodies, can be used for therapeutic, research or other applications. Animal cells, notably mammalian cells, are often used to produce proteins. Unfortunately, using animal cells causes the production process to be time consuming and costly.

Adding a chemical agent to a cell culture medium can increase cell productivity by inducing cells to produce a product, thereby increasing overall yield. The optimal agent to use varies depending on a number of factors, including the desired protein product and cell type. Similar factors also affect the amount of the chosen agent added and when the agent is added to the cell culture medium. Examples of agents are alkanoic acids or salts, urea derivatives, or dimethylsulphoxide (DMSO). Chemical agents, such as sodium butyrate, can have diverse effects on protein production. The addition of an agent can increase the specific productivity of the cells, but also have cytotoxic effects and can inhibit cell growth and viability.

As cells produce a protein, typically, the protein is secreted into the cell culture medium. The specific protein, however, is not the only matter in the medium; high molecular weight aggregates, acidic species, and other materials are also often in the medium, which can make the process of purification more laborious and costly. Techniques and methods are available to improve product quality, enabling more efficient protein purification; including, among others, altering the conditions of the bioreactor or using a different cell line. However, there nevertheless remains a need in the field for protein production techniques and methods that lead to improved purification processes.

Therefore, what is needed is a chemical agent that is added to the cell culture medium that can enhance the expression of a protein of interest while maintaining high cell viability. What is further needed is an agent that increases the product quality of the protein by decreasing the amount of high molecular weight aggregates and acidic species in the cell culture medium.

### SUMMARY OF THE INVENTION

In certain embodiments, the present disclosure relates to a process for enhanced production of a protein product. For example, in certain embodiments, the present invention provides methods of culturing host cells expressing a protein of interest in a medium comprising an anti-senescence compound such that overall production of the protein of interest is enhanced. In certain embodiments, such an anti-senescence compound comprises carnosine.

In certain embodiments, the present invention provides compositions that enhance production of a protein of interest. For example, in certain embodiments, the present invention provides a cell culture medium comprising an anti-senescence compound that enhances production of a protein of interest expressed in a host cell. In certain embodiments, such an anti-senescence compound comprises carnosine. In certain embodiments, genetically manipulated host cells are combined with an inoculum medium to form a cell culture medium, which is grown in a bioreactor. During a production run of the desired protein product, conditions of the bioreactor may be altered and/or supplements may be added in order to increase the productivity and/or maintain viability. Supplements may include a feed medium and/or one or more additives such as in the instant disclosure, carnosine and/or other anti-senescence compounds.

Mammalian host cells, for example, Chinese hamster ovary (CHO) cells, can experience a reduction in viability nearing the end of a production run in a bioreactor. It has been discovered that the addition of an anti-senescence agent, such as carnosine and analogs thereof, to a cell culture medium helps maintain a higher viable cell number and cell viability until the protein is harvested.

In addition, methods for increasing productivity, such as altering the temperature after the growth phase and/or during the production phase of the production run may be used in accordance with the present invention. To give but one example, during the production of a protein product, specifically the antibody for growth differentiation factor-8 (GDF-8), the temperature was shifted downwards to help initiate and increase the productivity. In certain embodiments, addition of an anti-senescence compound such as carnosine helps increase the productivity of the cell culture. In certain embodiments, an anti-senescence compound may be added before, during and/or after such a temperature shift.

It has also been discovered that the addition of an anti-senescence compound such as carnosine to a cell culture medium increases the overall quality of protein product. During the production of the protein, high molecular weight aggregates, along with other unwanted species, are in the cell culture medium. The addition of carnosine decreases the amount of high molecular aggregates and increases product quality. In certain embodiments, addition of an anti-senescence compound other than carnosine decreases accumulation of such high molecular weight aggregates and improves product quality. In certain embodiments, carnosine is added in combination with one or more additional anti-senescence compounds.

The concentration of anti-senescence compound (e.g., carnosine) added to the cell culture medium can vary depending on many factors of the process, including, for example, the cell type, the desired product, and the conditions of the bioreactor, among others. Also, carnosine can be substituted with its analogues; acetyl-carnosine, homo-carnosine, anserine, and β-alanine. In certain embodiments, carnosine is provided in combination with one or more other anti-senescence compounds. In certain embodiments, the concentration of anti-senescence agent (e.g., carnosine) in a cell culture medium is about 5 mM to about 100 mM. In certain embodiments, the concentration is about 10 mM to about 40 mM. In certain embodiments, the concentration is about 20 mM.

Any suitable culture procedures and inoculum medium may be used to culture the cells in the process of protein production. Both serum and serum free media may be used. In addition, culturing methods may be used to culture the cells as appropriate for the specific cell type and protein product. Such procedures are known and understood by those of ordinary skill in the cell culture art.

Other features and advantages of the disclosure will be apparent from the following description, and from the claims.

### BRIEF DESCRIPTION OF THE DRAWINGS

Figure 1a. Effect of Carnosine on Acidic Peaks for MYO-29.
Figure 1b. Effect of Carnosine on High Molecular Weight Aggregates.
Figure 1c. Effect of Carnosine Additions on Different Days on Acidic Peaks.
Figure 1d. Effect of Carnosine Additions on Different Days on High Molecular Weight Aggregates.
Figure 2a. Effect of Carnosine on Daily Viable Cell Density.
Figure 2b. Effect of Carnosine on Daily Cell Viability.
Figure 2c. Effect of Carnosine on Daily Titer.
Figure 2d. Effect of Carnosine on Cumulative Specific Cellular Productivity.
Figure 2e. Effect of Carnosine on High Molecular Weight Aggregates.
Figure 3a. Effect of Different Concentrations of Carnosine on Viable Cell Density.
Figure 3b. Effect of Different Concentrations of Carnosine on Daily Cell Viability.
Figure 3c. Effect of Different Concentrations of Carnosine on Daily Titer.
Figure 3d. Effect of Different Concentrations of Carnosine on Cumulative Specific Cellular Productivity.
Figure 3e. Effect of Different Concentrations of Carnosine on High Molecular Weight Aggregates.

### DEFINITIONS

Following long-standing convention, the terms "a" and "an" mean "one or more" when used in this application, including the claims. Even though the invention has been described with a certain degree of particularity, it is evident that many alternatives, modifications, and variations will be apparent to those skilled in the art in light of the disclosure. Accordingly, it is intended that all such alternatives, modifications, and variations, which fall within the spirit and scope of the invention, be embraced by the defined claims.

The term "anti-senescence compound" as used herein refers to any agent or compound that, when added to a cell culture, promotes viability, growth, and/or lifespan of a cell grown therein. In certain embodiments, use of such an anti-senescence compound in cell culture results in increased titer, increased cell specific productivity, increased cell viability, increased integrated viable cell density, decreased accumulation of high molecular weight aggregates, and/or decreased accumulation of acidic species than would be observed under otherwise identical culture conditions that lack the anti-senescence compound. Non-limiting examples of anti-senescence compounds that may be used in accordance with methods and compositions of the present invention include carnosine, acetyl-carnosine, homocarnosine, anserine, and β-alanine. In certain embodiments, two or more anti-senescence compounds may be used in accordance with compositions and methods of the present invention.

The phrase "host cell" refers to cells which are capable of being genetically manipulated and/or are capable of growth and survival in a cell culture medium. Typically, the cells can express a large quantity of an endogenous or heterologous protein of interest and can either retain the protein or secrete it into the cell culture medium

Host cells are typically "mammalian cells," which comprise the nonlimiting examples of vertebrate cells, including baby hamster kidney (BHK), Chinese hamster ovary (CHO), human kidney (293), normal fetal rhesus diploid (FRhL-2), and murine myeloma (e.g., SP2/0 and NS0) cells. One of ordinary skill in the art will be aware of other host cells that may be used in accordance with methods and compositions of the present invention.

The term "cell culture medium" refers to a solution containing nutrients to support cell survival under conditions in which cells can grow and produce a desired protein. The phrase "inoculation medium" or "inoculum medium" refer to a solution or substance containing nutrients in which a culture of cells is initiated. In certain embodiments a "feed medium" contains similar nutrients as the inoculation medium, but is a solution or substance with which the cells are fed after initiation of the culture. In certain embodiments, a feed medium contains one or more components not present in an inoculation medium. In certain embodiments, a feed medium lacks one or more components present in an inoculation medium. A person of ordinary skill in the cell culture art will know without undue experimentation what components make-up the inoculation and feed mediums. Typically, these solutions provide essential and non-essential amino acids, vitamins, energy sources, lipids, and trace elements required by a cell for growth and survival. In certain embodiments, an inoculation medium, a feed medium or both comprise an anti-senescence compound.

The term "cell culture characteristic" as used herein refers to an observable and/or measurable characteristic of a cell culture. Methods and compositions of the present invention are advantageously used to improve one or more cell culture characteristics. In certain embodiments, improvement of a cell culture characteristic comprises increasing the magnitude of a cell culture characteristic. In certain embodiments, improvement of a cell culture characteristic comprises decreasing the magnitude of a cell culture characteristic. As non-limiting examples, a cell culture characteristic may be titer, cell specific productivity, cell viability, integrated viable cell density, accumulation of high molecular weight aggregates, and/or accumulation of acidic species. One of ordinary skill in the art will be aware of other cell culture characteristics that may be improved using methods and compositions of the present invention.

The term "defined medium" as used herein refers to a medium in which the composition of the medium is both known and controlled. Defined media do not contain complex additives such as serum or hydrolysates that contain unknown and/or uncontrolled components.

The term "complex medium" as used herein refers to a medium contains at least one component whose identity or quantity is either unknown or uncontrolled.

The phrase "cell line" refers to, generally, primary host cells that express a protein of interest. In some embodiments, the cells have been transfected with exogenous DNA coding for a desired protein and/or containing control sequences that activate expression of linked sequences, whether endogenous or heterologous. In certain embodiments, cells derived from such genetically modified cells form a cell line and are placed in a cell culture medium to grow and produce the protein product. In certain embodiments, a cell line comprises primary host cells that have not been transfected with exogenous DNA and express an endogenous protein of interest.

The "growth phase" of a cell culture medium refers to the period when the cells are undergoing rapid division and growing exponentially, or close to exponentially. Typically, cells are cultured in conditions optimized for cell growth for generally 1-4 days. Growth phase conditions may include a temperature at about 35°C to 42°C, generally about 37°C. The length of the growth phase and the culture conditions in the growth phase can vary but are generally known to a person of ordinary skill in the cell culture art. In certain embodiments, a cell culture medium in a growth phase is supplemented with a feed medium.

The "transition phase" occurs during the period when the cell culture medium is being shifted from conditions consistent with the growth phase to conditions consistent with the production phase. During the transition phase, factors like temperature, among others, are often changed. In certain embodiments, a cell culture medium in a transition phase is supplemented with a feed medium.

The "production phase" occurs after both the growth phase and the transition phase. The exponential growth of the cells has ended and protein production is the principal objective. The cell culture medium can be supplemented to initiate production. In certain embodiments, a cell culture medium in a production phase is supplemented with a feed medium. In addition, the temperature of the cell culture medium during the production phase may be lower, generally, than during the growth phase, which typically encourages production. The production phase continues until a desired endpoint is achieved.

The phrase "viable cell density" refers to the total number of cells that are surviving in the cell culture medium in a particular volume, generally per ml. The phrase "cell viability" refers to number of cells, which are alive compared to the total number of cells, both dead and alive, expressed as a percentage.

"Integrated Viable Cell Density", "IVCD": The terms "integrated viable cell density" or "IVCD" as used herein refer to the average density of viable cells over the course of the culture multiplied by the amount of time the culture has run. When the amount of protein produced is proportional to the number of viable cells present over the course of the culture, integrated viable cell density is a useful tool for estimating the amount of protein produced over the course of the culture.

The term "high molecular weight aggregates" refers to generally mis-folded proteins or an improper association of at least two polypeptides. The association may arise by any method including, but not limited to, covalent, non-covalent, disulfide, or nonreducible cross linking. In certain embodiments, methods and compositions of the present invention are advantageously utilized to reduce the accumulation of high molecular weight aggregates.

The phrase "antioxidant" refers to a compound that can prevent oxidative damage to lipids, proteins, DNA and other essential macromolecules by blocking free radicals.

As used herein, the term "antibody" includes a protein comprising at least one, and typically two, VH domains or portions thereof, and/or at least one, and typically two, VL domains or portions thereof. In certain embodiments, the antibody is a tetramer of two heavy immunoglobulin chains and two light immunoglobulin chains, wherein the heavy and light immunoglobulin chains are inter-connected by, e.g., disulfide bonds. The antibodies, or a portion thereof, can be obtained from any origin, including, but not limited to, rodent, primate (e.g., human and non-human primate), camelid, as well as recombinantly produced, e.g., chimeric, humanized, and/or in vitro generated, as described in more detail herein.

Examples of binding fragments encompassed within the term "antigen-binding fragment" of an antibody include, but are not limited to, (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment, which consists of a VH domain; (vi) a camelid or camelized variable domain; (vii) a single chain Fv (scFv); (viii) a bispecific antibody; and (ix) one or more fragments of an immunoglobulin molecule fused to an Fc region. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see, e.g., Bird et al. (1988) Science 242:423-26; Huston et al. (1988) Proc. Natl. Acad. Sci. U.S.A. 85:5879-83). Such single chain antibodies are also intended to be encompassed within the term "antigen-binding fragment" of an antibody. These fragments may be obtained using conventional techniques known to those skilled in the art, and the fragments are evaluated for function in the same manner as are intact antibodies.

The "antigen-binding fragment" can, optionally, further include a moiety that enhances one or more of, e.g., stability, effector cell function or complement fixation. For example, the antigen binding fragment can further include a pegylated moiety, albumin, or a heavy and/or a light chain constant region.

Other than "bispecific" or "bifunctional" antibodies, an antibody is understood to have each of its binding sites identical. A "bispecific" or "bifunctional antibody" is an artificial hybrid antibody having two different heavy/light chain pairs and two different binding sites. Bispecific antibodies can be produced by a variety of methods including fusion of hybridomas or linking of Fab' fragments. See, e.g., Songsivilai & Lachmann, Clin. Exp. Immunol. 79:315-321 (1990); Kostelny et al., J. Immunol. 148, 1547-1553 (1992).

Numerous methods known to those skilled in the art are available for obtaining antibodies or antigen-binding fragments thereof. For example, monoclonal antibodies may be produced by generation of hybridomas in accordance with known methods. Hybridomas formed in this manner are typically screened using standard methods, such as enzyme-linked immunosorbent assay (ELISA) and surface plasmon resonance (Biacore™) analysis, to identify one or more hybridomas that produce an antibody that specifically binds with a specified antigen. Any form of the specified antigen may be used as the immunogen, e.g., recombinant antigen, naturally occurring forms, any variants or fragments thereof, as well as antigenic peptide thereof.

One exemplary method of making antibodies includes screening protein expression libraries, e.g., phage or ribosome display libraries. Phage display is described, for example, in Ladner et al., U.S. Patent No. 5,223,409; Smith (1985) Science 228:1315-1317; WO 92/18619; WO 91/17271; WO 92/20791; WO 92/15679; WO 93/01288; WO 92/01047; WO 92/09690; and WO 90/02809.

In addition to the use of display libraries, the specified antigen can be used to immunize a non-human animal, e.g., a rodent, e.g., a mouse, hamster, or rat. In certain embodiments, the non-human animal includes at least a part of a human immunoglobulin gene. For example, it is possible to engineer mouse strains deficient in mouse antibody production with large fragments of the human Ig loci. Using the hybridoma technology, antigen-specific monoclonal antibodies derived from the genes with the desired specificity may be produced and selected. See, e.g., XENOMOUSE™, Green et al. (1994) Nature Genetics 7:13-21, US 2003-0070185, WO 96/34096, published Oct. 31, 1996, and PCT Application No. PCT/US96/05928, filed Apr. 29, 1996.

In certain embodiments, a monoclonal antibody is obtained from the non-human animal, and then modified, e.g., humanized, deimmunized, chimeric, may be produced using recombinant DNA techniques known in the art. A variety of approaches for making chimeric antibodies have been described. See e.g., Morrison et al., Proc. Natl. Acad. Sci. U.S.A. 81:6851, 1985; Takeda et al., Nature 314:452, 1985, Cabilly et al., U.S. Patent No. 4,816,567; Boss et al., U.S. Patent No. 4,816,397; Tanaguchi et al., European Patent Publication EP171496; European Patent Publication 0173494, United Kingdom Patent GB 2177096B. Humanized antibodies may also be produced, for example, using transgenic mice that express human heavy and light chain genes, but are incapable of expressing the endogenous mouse immunoglobulin heavy and light chain genes. Winter describes an exemplary CDR-grafting method that may be used to prepare the humanized antibodies described herein (U.S. Patent No. 5,225,539). All of the CDRs of a particular human antibody may be replaced with at least a portion of a non-human CDR, or only some of the CDRs may be replaced with non-human CDRs. It is only necessary to replace the number of CDRs required for binding of the humanized antibody to a predetermined antigen.

Humanized antibodies or fragments thereof can be generated by replacing sequences of the Fv variable domain that are not directly involved in antigen binding with equivalent sequences from human Fv variable domains. Exemplary methods for generating humanized antibodies or fragments thereof are provided by Morrison (1985) Science 229:1202-1207; by Oi et al. (1986) BioTechniques 4:214; and by US 5,585,089; US 5,693,761; US 5,693,762; US 5,859,205; and US 6,407,213. Such methods include isolating, manipulating, and expressing the nucleic acid sequences that encode all or part of immunoglobulin Fv variable domains from at least one of a heavy or light chain. Such nucleic acids may be obtained from a hybridoma producing an antibody against a predetermined target, as described above, as well as from other sources. A recombinant DNA encoding a humanized antibody molecule can then be cloned into an appropriate expression vector.

In certain embodiments, a humanized antibody is optimized by the introduction of conservative substitutions, consensus sequence substitutions, germline substitutions and/or backmutations. Such altered immunoglobulin molecules can be made by any of several techniques known in the art, (e.g., Teng et al., Proc. Natl. Acad. Sci. U.S.A., 80: 7308-7312, 1983; Kozbor et al., Immunology Today, 4: 7279, 1983; Olsson et al., Meth. Enzymol., 92: 3-16, 1982), and may be made according to the teachings of PCT Publication WO92/06193 or EP 0239400).

An antibody or fragment thereof may also be modified by specific deletion of human T cell epitopes or "deimmunization" by the methods disclosed in WO 98/52976 and WO 00/34317. Briefly, the heavy and light chain variable domains of an antibody can be analyzed for peptides that bind to MHC Class II; these peptides represent potential T-cell epitopes (as defined in WO 98/52976 and WO 00/34317). For detection of potential T-cell epitopes, a computer modeling approach termed "peptide threading" can be applied, and in addition a database of human MHC class II binding peptides can be searched for motifs present in the VH and VL sequences, as described in WO 98/52976 and WO 00/34317. These motifs bind to any of the 18 major MHC class II DR allotypes, and thus constitute potential T cell epitopes. Potential T-cell epitopes detected can be eliminated by substituting small numbers of amino acid residues in the variable domains, or preferably, by single amino acid substitutions. Typically, conservative substitutions are made. Often, but not exclusively, an amino acid common to a position in human germline antibody sequences may be used. Human germline sequences, e.g., are disclosed in Tomlinson, et al. (1992) J. Mol. Biol. 227:776-798; Cook, G. P. et al. (1995) Immunol. Today Vol. 16 (5): 237-242; Chothia, D. et al. (1992) J. Mol. Biol. 227:799-817; and Tomlinson et al. (1995) EMBO J. 14:4628-4638. The V BASE directory provides a comprehensive directory of human immunoglobulin variable region sequences (compiled by Tomlinson, I.A. et al. MRC Centre for Protein Engineering, Cambridge, UK). These sequences can be used as a source of human sequence, e.g., for framework regions and CDRs. Consensus human framework regions can also be used, e.g., as described in US 6,300,064.

In certain embodiments, an antibody can contain an altered immunoglobulin constant or Fc region. For example, an antibody produced in accordance with the teachings herein may bind more strongly or with more specificity to effector molecules such as complement and/or Fc receptors, which can control several immune functions of the antibody such as effector cell activity, lysis, complement-mediated activity, antibody clearance, and antibody half-life. Typical Fc receptors that bind to an Fc region of an antibody (e.g., an IgG antibody) include, but are not limited to, receptors of the FcγRI, FcγRII, and FcγRIII and FcRn subclasses, including allelic variants and alternatively spliced forms of these receptors. Fc receptors are reviewed in Ravetch and Kinet, Annu. Rev. Immunol 9:457-92, 1991; Capel et al., Immunomethods 4:25-34,1994; and de Haas et al., J. Lab. Clin. Med. 126:330-41, 1995).

The phrase "bioreactor" refers to a vessel in which a cell culture medium can be contained and internal conditions of which can be controlled during the culturing period, e.g., pH and temperature.

A "fed batch culture" refers to a method of culturing cells in which cells are first inoculated in a bioreactor with an inoculum medium. The cell culture medium is then supplemented at one or more points throughout the production run with a feed medium containing nutritional components and/or other supplements.

A "batch culture" refers to a method of culturing cells in which cells are inoculated in a bioreactor with all the necessary nutrients and supplements for the entirety of the production run. No nutrient additions are made to the cell culture medium throughout the duration of the production.

A "perfusion culture" refers to a method of culturing cells that is different from a batch or fed-batch culture method, in which the culture is not terminated, or is not necessarily terminated, prior to isolating and/or purifying an expressed protein of interest, and in which new nutrients and other components are periodically or continuously added to the culture, during which the expressed protein is periodically or continuously harvested. The composition of the added nutrients may be changed during the course of the cell culture, depending on the needs of the cells, the requirements for optimal protein production, and/or any of a variety of other factors known to those of ordinary skill in the art.

The phrase "expression" refers to the transcription and the translation that occurs within a host cell. The level of expression relates, generally, to the amount of protein being produced by the host cell.

The phrases "protein" or "protein product" refer to one or more chains of amino acids. As used herein, the term "protein" is synonymous with "polypeptide" and, as is generally understood in the art, refers to at least one chain of amino acids liked via sequential peptide bonds. In certain embodiments, a "protein of interest" is a protein encoded by an exogenous nucleic acid molecule that has been transformed into a host cell. In certain embodiments, a "protein of interest" is a protein encoded by a nucleic acid molecule that is endogenous to the host cell. In certain embodiments, expression of such an endogenous protein of interest is altered by transfecting a host cell with an exogenous nucleic acid molecule that may, for example, contain one or more regulatory sequences and/or encode a protein that enhances expression of the protein of interest. Methods and compositions of the present invention may be used to produce any protein of interest, including, but not limited to proteins having therapeutic, pharmaceutical, diagnostic, agricultural, and/or any of a variety of other properties that are useful in commercial, experimental and/or other applications. In certain embodiments, proteins produced using methods and/or compositions of the present invention may be processed and/or modified. For example, a protein to be produced in accordance with the present invention may be glycosylated.

"Cell specific productivity", and the like, refer to the specific, as in per cell, product expression rate. The cell specific productivity is generally measured in micrograms of protein produced per 10⁶ cells per day or in picograms of protein produced per 10⁶ cells per day.

The term "titer" as used herein refers to the total amount of recombinantly expressed protein produced by a cell culture in a given amount of medium volume. Titer is typically expressed in units of milligrams or micrograms of protein per milliliter of medium.

. One of skill in the art will recognize that the methods disclosed herein may be used to culture many of the well-known mammalian cells routinely used and cultured in the art, i.e., the methods disclosed herein are not limited to use with only the instant disclosure.

### DETAILED DESCRIPTION OF CERTAIN EMBODIMENTS OF THE INVENTION

It has been discovered that using an anti-senescence compound such as carnosine modifies the viability and productivity of a cell culture. For example, addition of carnosine maintains cell viability, and improves productivity of cells, and improves product quality of the desired protein product. Carnosine is an antioxidant and an anti-senescence compound that is also a naturally occurring dipeptide present at high levels (up to 20 mM) in muscle and nerve tissues in animals. Being an antioxidant, carnosine also is a free radical scavenger and glycation inhibitor. Generally, carnosine transforms reactive species into non-reactive species thereby protecting proteins, DNA, and other essential macromolecules. As an anti-senescence compound, carnosine can extend the lifespan of human diploid fibroblasts and human fetal lung (primary cell lines) at a concentration of 20 mM in culture media. The present invention encompasses the surprising finding that it is advantageous to use an anti-senescence compound (including, but not limited to, carnosine) in cell culture to produce a protein of interest. In certain embodiments, using such an anti-senescence compound in cell culture to produce a protein of interest results in one or more improved cell culture characteristics including, but not limited to, increased titer, increased cell specific productivity, increased cell viability, increased integrated viable cell density, decreased accumulation of high molecular weight aggregates, and/or decreased accumulation of acidic species.

It has been demonstrated that carnosine is cytotoxic to human or rodent transformed and neoplastic cells in Minimal Essential Medium (MEM, Sigma), which has lower glucose levels, but not in Dulbecco's Modified Eagle's Medium (DMEM, Sigma), which contains 1 mM pyruvate. (Holliday et al, Biochemistry (Moscow), 65:843-848,846). In addition, dialyzed fetal calf serum with low molecular weight compounds removed increased the cytotoxic effects of carnosine. *Id.* It was also determined that 1mM oxaloacetate and 1 mM of α-ketoglutarate had comparable effects as pyruvate, neither of which are components of the inoculum or feed mediums used with the carnosine examples. *Id*. Sodium pyruvate, however, is an original component in the inoculum medium at a concentration of 0.5 mM, not for carnosine additions but rather to better mimic in vivo conditions in a bioreactor system and as a potential alternate energy source. The inoculum medium is also serum free, which would imply that carnosine would have cytotoxic effects. According to the reference, the addition of carnosine to a cell culture medium would have similar cytotoxic effects as seen in the MEM medium in *Holliday.* By utilizing methods and/or compositions of the present invention, such cytotoxicity is reduced or eliminated and cell viability and protein production are improved.

In certain embodiments, carnosine is provided in a cell culture medium at a concentration of between about 5 mM and about 100 mM. In certain embodiments, carnosine is provided in a cell culture medium at a concentration of about 10 mM to about 40 mM, for example at a concentration of about 20 mM. In certain embodiments, carnosine is provided in a cell culture medium at a concentration of about 5, 6, 7, 8, 9, 10, 11, 12, 13, 14, 15, 16,17,18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100 mM, or higher. In certain embodiments, such concentrations of carnosine are achieved in cell culture by adding carnosine at multiple times during the cell culture process, for example, in one or more feed media. The concentration of carnosine utilized depends on the cell culture medium and the cell line being used, among other factors, including the desired effects being sought on the cell line or product. Analogs of carnosine, e.g. acetyl-carnosine, homo-carnosine, anserine, and β-alanine, may also be provided in a cell culture medium for a similar effect. One or more of these analogs may be provided in a cell culture medium. In certain embodiments, such analogs are provided in a cell culture medium that lacks carnosine. In certain embodiments, such analogs are provided in a cell culture medium in combination with carnosine. In certain embodiments, such analogs are provided at a concentration of about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 38, 37, 38, 39, 40, 41, 42, 43, 44, 45, 45, 47, 48, 49, 50, 55, 60, 65, 70, 75, 80, 85, 90, 95, 100 mM, or higher.

In certain embodiments, in order to produce a protein of interest, initially, host cells are transfected or transformed with exogenous DNA coding for a protein to supply transformed cells, which constitutively produce the desired protein product. In certain embodiments, a nucleic acid molecule introduced into the cell encodes the protein desired to be expressed according to the present invention. In certain embodiments, a nucleic acid molecule contains a regulatory sequence or encodes a gene product that induces or enhances the expression of the desired protein by the cell. As a non-limiting example, such a gene product may be a transcription factor that increases expression of the protein of interest.

In certain embodiments, a nucleic acid that directs expression of a protein is stably introduced into the host cell. In certain embodiments, a nucleic acid that directs expression of a protein is transiently introduced into the host cell. One of ordinary skill in the art will be able to choose whether to stably or transiently introduce the nucleic acid into the cell based on experimental, commercial or other needs.

A gene encoding a protein of interest may optionally be linked to one or more regulatory genetic control elements. In some embodiments, a genetic control element directs constitutive expression of the protein. In some embodiments, a genetic control element that provides inducible expression of a gene encoding the protein of interest can be used. Use of an inducible genetic control element (e.g., an inducible promoter) allows for modulation of the production of the protein in the cell. Non-limiting examples of potentially useful inducible genetic control elements for use in eukaryotic cells include hormone- regulated elements (see e.g., Mader, S. and White, J.H., Proc. Natl. Acad. Sci. USA 90:5603-5607, 1993), synthetic ligand-regulated elements (see, e.g. Spencer, D.M. et al., Science 262:1019-1024, 1993) and ionizing radiation-regulated elements (see e.g., Manome, Y. et al., Biochemistry 32:10607-10613, 1993; Datta, R. et al., Proc. Natl. Acad. Sci. USA 89:10149-10153, 1992). Additional cell-specific or other regulatory systems known in the art may be used in accordance with methods and compositions described herein.

Any host cell susceptible to cell culture, and to expression of proteins, may be utilized in accordance with the present invention. The host cells are generally mammalian cells, more particularly animal cells, such as Chinese hamster ovary (CHO) cells. Other non-limiting examples of mammalian cells that may be used in accordance with the present invention include BALB/c mouse myeloma line (NSO/I, ECACC No: 85110503); human retinoblasts (PER.C6 (CruCell, Leiden, The Netherlands)); monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); baby hamster kidney cells (BHK, ATCC CCL 10); Chinese hamster ovary cells +/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); monkey kidney cells (CV1 ATCC CCL 70); African green monkey kidney cells (VERO-76, ATCC CRL-1 587); human cervical carcinoma cells (HeLa, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68 (1982)); MRC 5 cells; FS4 cells; and a human hepatoma line (Hep G2).

Any protein that is expressible in a host cell may be produced in accordance with methods and compositions of the present invention. The protein may be expressed from a gene that is endogenous to the host cell, or from a heterologous gene that is introduced into the host cell. The protein may be one that occurs in nature, or may alternatively have a sequence that was engineered or selected by the hand of man. A protein to be produced may be assembled from protein fragments that individually occur in nature. Additionally or alternatively, the engineered protein may include one or more fragments that are not naturally occurring.

In certain embodiments, methods and compositions of the present invention are employed to express a pharmaceutically or commercially relevant enzyme, receptor, antibody, hormone, regulatory factor, antigen, binding agent, etc. In the instant example, the genetically manipulated cells produce an antibody against growth differentiation factor-8 (GDF-8). Nonlimiting illustrative embodiments are termed Myo29, Myo28, and Myo22. These exemplary embodiments are provided in the form of human IgG isotopes. The nonlimiting examples disclosed use MYO29 covered by patent # WO 2004/037861 entitled Neutralizing Antibodies Against GDF-8 and Uses Thereof, and is herein incorporated by reference. One of ordinary skill in the art will be aware of other useful and/or desirable proteins that may be expressed in accordance with methods and compositions of the present invention.

Cell lines may be cultured using a variety of techniques to produce the desired protein product. The cell culture can be done on a small or large scale, depending on the purpose of the cell culture medium or use of the product. For example, cells may be grown in a bioreactor. In certain embodiments, the volume of the bioreactor is at least 1 liter and may be 10, 100, 250, 500, 1,000, 2,500, 5,000, 8,000, 10,000, 12,000 liters or more, or any volume in between. In addition, bioreactors that may be used include, but are not limited to, a stirred tank bioreactor, fluidized bed reactor, hollow fiber bioreactor, or roller bottle. The systems can also operate either in a batch, fed-batch, or continuous/perfusion mode. The bioreactor and mode in which to control and monitor the cell culture medium will be known to one of ordinary skill in the cell culture art. In the instant example, the system utilized is a stirred tank bioreactor and operated in fed-batch mode.

The bioreactor is generally seeded with an inoculum medium and a chosen cell line, for example a MYO-29 cell line, which may be transfected to stably express and produce the desired protein product. Commercially available medium such as Minimal Essential Medium (MEM, Sigma), Ham's F10 (Sigma), or Dulbecco's Modified Eagle's Medium (DMEM, Sigma) may be used as the base medium. These base mediums may then be supplemented with amino acids, vitamins, trace elements, and/or other components to produce the inoculum or feed mediums used during the production run. In certain embodiments, a base medium is altered to permit robust growth of cells, to increase cell viability, to increase cell productivity, to increase integrated viable cell density, and/or to improve the quality of the produced protein in the presence of carnosine. For example, a base medium may be supplemented with pyruvate, oxaloacetate and/or α-ketoglutarate. One of ordinary skill in the art will be able to alter a base medium for use with methods and compositions of the present invention without undue experimentation.

In certain embodiments, cells are cultured in any of a variety of chemically defined media containing an anti-senescence compound, wherein the components of the media are both known and controlled. For example, defined media typically do not contain complex additives such as serum or hydrolysates. In certain embodiments, cells are cultured in any of a variety of complex media containing an anti-senescence compound, in which not all components of the medium are known and/or controlled. In certain embodiments, such an anti-senescence compound comprises carnosine.

The conditions of the bioreactor are controlled typically, with the pH set between about 6.5 to about 7.5. The pH is adjusted using an acid, generally CO₂, or a base, such as sodium bicarbonate. The dissolved oxygen is controlled between about 5 and 90% of air saturation, and the temperature is held between 30°C to 42°C, during the growth phase. A person of ordinary skill in the cell culture art can modify the conditions of the bioreactor based upon the cell line and methods being employed to achieve the desired results without undue experimentation.

Compositions and methods of the present invention may be used with any cell culture method or system that is amenable to the expression of proteins. For example, cells expressing a protein of interest may be grown in batch or fed-batch cultures, wherein the culture is terminated after sufficient expression of the protein, after which the expressed protein is harvested and optionally purified. Alternatively, cells expressing a protein of interest may be grown in perfusion cultures, wherein the culture is not terminated and new nutrients and other components are periodically or continuously added to the culture, during which the expressed protein is periodically or continuously harvested.

After the cells are seeded they go through a growth phase during which the number of cells generally increases exponentially. During the growth phase, the temperature or temperature range of the cell culture will be selected based primarily on the temperatures or range of temperatures at which the cell culture remains viable, at which a high level of protein is produced, at which production or accumulation of metabolic waste products is minimized, and/or any combination of these or other factors deemed important by the practitioner. As one non-limiting example, CHO cells grow well and produce high levels or protein at approximately 37°C. In general, most mammalian cells grow well and/or can produce high levels or protein within a range of about 25°C to 42°C, although methods taught by the present disclosure are not limited to these temperatures. Certain mammalian cells grow well and/or can produce high levels of protein within the range of about 35°C to 40°C. In certain embodiments, the cell culture is grown at a temperature of 20, 21, 22, 23, 24, 25, 26, 27, 28, 29, 30, 31, 32, 33, 34, 35, 36, 37, 38, 39, 40, 41, 42, 43, 44, or 45°C at one or more times during the growth phase. Those of ordinary skill in the art will be able to select appropriate temperature or temperature range in which to grow cells, depending on the needs of the cells and the production requirements of the practitioner.

Following the growth phase is a transition phase during which the cells adapt to any changes occurring in the surroundings, such as a temperature change. The changes occurring are typically parameters for the production phase. In the instant examples, on day 4, the temperature decreased from about 37°C to about 31°C. The temperature shift, however, can occur more than once and does not need to necessarily go in the downward direction. Moreover, the transition phase and the temperature shift can occur on any day during the production run. Although most methods of production include multi-phase processes, carnosine also may be utilized in a single-phase process.

When shifting the temperature of the culture, the temperature shift may be relatively gradual. For example, it may take several hours or days to complete the temperature change. Alternatively, the temperature shift may be relatively abrupt. The temperature may be steadily increased or decreased during the culture process. Alternatively, the temperature may be increased or decreased by discrete amounts at various times during the culture process. The subsequent temperature(s) or temperature range(s) may be lower than or higher than the initial or previous temperature(s) or temperature range(s). One of ordinary skill in the art will understand that multiple discrete temperature shifts are encompassed in these embodiments. For example, the temperature may be shifted once (either to a higher or lower temperature or temperature range), the cells maintained at this temperature or temperature range for a certain period of time, after which the temperature may be shifted again to a new temperature or temperature range, which may be either higher or lower than the temperature or temperature range of the previous temperature or temperature range. The temperature of the culture after each discrete shift may be constant or may be maintained within a certain range of temperatures.

Finally, there is the production phase where the cell number does not substantially increase, but rather the cells produce the desired protein product. One of ordinary skill in the art will understand, however, that in certain embodiments, cells may continue to grow and increase in number during the production phase. During this phase the environment of the bioreactor is controlled at conditions in which the cells are more likely to be productive. For example, the temperature is generally held at a temperature different than that of the growth phase, which is conducive to the production of a protein product, e.g. 31°C. Throughout the production run, the cells may be fed a feed medium containing nutrients and supplements the cells may need. For example, in certain cases, it may be beneficial or necessary to supplement the cell culture during the subsequent production phase with nutrients or other medium components that have been depleted or metabolized by the cells. As non-limiting examples, it may be beneficial or necessary to supplement the cell culture with hormones and/or other growth factors, particular ions (such as sodium, chloride, calcium, magnesium, and phosphate), buffers, vitamins, nucleosides or nucleotides, trace elements (inorganic compounds usually present at very low final concentrations), amino acids, lipids, or glucose or other energy source. These supplementary components may all be added to the cell culture at one time, or they may be provided to the cell culture in a series of additions. In certain embodiments, an anti-senescence compound is provided in a feed medium at one or more times during the production phase.

According to certain embodiments, use of an anti-senescence compound, e.g. carnosine, in the cell culture medium during the production phase, whether provided in an inoculation medium or a feed medium, increases cell viability and/or specific protein production, thus improving the overall yield of produced protein.

Aspects of a protein production process are determined by one of ordinary skill in the cell culture art. The parameters such as seed density, duration of the production culture; operating conditions during harvest, among others, including those mentioned above are functions of the cell line and the cell culture medium. Therefore, the parameters can be determined without undue experimentation by a person of ordinary skill in the cell culture art.

As with the temperature or temperature range during the growth phase, the temperature or temperature range of the cell culture during the production phase will be selected based primarily on the temperature or temperature range at which the cell culture remains viable, at which a high level of protein is produced, at which production or accumulation of metabolic waste products is minimized, and/or any combination of these or other factors deemed important by the practitioner. In general, most mammalian cells remain viable and produce high levels or protein within a range of about 25°C to 42°C, although methods taught by the present disclosure are not limited to these temperatures. In certain embodiments, mammalian cells remain viable and produce high levels or protein within a range of about 25°C to 35°C. In certain embodiments, the cell culture is grown at a temperature of 20, 21, 22, 23, 24, 25, 26, 27, 28, 29, 30, 31, 32, 33, 34, 35, 36, 37, 38, 39, 40, 41, 42, 43, 44, or 45°C at one or more times during the production phase. Those of ordinary skill in the art will be able to select appropriate temperature(s) or temperature range(s) in which to grow cells during the production phase, depending on the particular needs of the cells and the particular production requirements of the practitioner. The cells may be grown for any amount of time, depending on the needs of the practitioner and the requirement of the cells themselves.

In certain embodiments, batch or fed-batch cultures are terminated once the culture achieves one or more relevant culture conditions, as determined by the needs of the practitioner. In certain embodiments, batch or fed-batch cultures are terminated once the expressed protein reaches a sufficiently high titer, once the cell density reaches a sufficiently high level, once the expressed protein reaches a sufficiently high cell density, and/or to prevent undesirable production or accumulation of metabolic waste products (e.g., lactate and/or ammonium). One of ordinary skill in the art will be aware of other relevant culture conditions that may be used to determine when a batch or fed-batch culture should be terminated, based on experimental, commercial, and/or other considerations.

In certain embodiments, following a production run, the protein product is recovered from the cell culture medium and further isolated using traditional separation techniques. For example, the protein may initially be separated by centrifugation, retaining the supernatant containing the protein. Additionally or alternatively, the protein product may be bound to the surface of the host cell. In such embodiments, the media is removed and the host cells expressing the protein are lysed as a first step in the purification process. Lysis of mammalian host cells can be achieved by any number of means known to those of ordinary skill in the art, including physical disruption by glass beads and exposure to high pH conditions.

Using conventional protein purification methods, the protein may be additionally isolated. Methods by which to isolate and purify the desired protein product are known within the cell culture art. Specific methods depend on the cell line used and the product sought.

The anti-senescence compound, e.g. carnosine, may be added to the culture medium at a time optimal for the specific cell culture process. For the instant examples, the addition of carnosine occurs after the growth phase is substantially complete and is in the transition phase. During the addition, the cell culture medium is adapting to the new temperature resulting from the temperature shift. The transition phase is generally when agents are added to help initiate the production phase. Carnosine, however, may be added at any point during the production run that generates optimal results, including the growth phase and the production phase. Carnosine may also be added in combination with other components, such as a feed medium. In certain embodiments, an anti-senescence compound is provided in an inoculation medium and is present in the cell culture during the entire cell culture process. In certain embodiments, two or more anti-senescence compounds are provided in the cell culture medium. In certain embodiments, two or more anti-senescence compounds are provided in an inoculation medium and are present in the cell culture during the entire cell culture process. In certain embodiments, two or more anti-senescence compounds are provided, wherein one anti-senescence compound is provided in an inoculation medium and is present in the cell culture during the entire cell culture process, while another anti-senescence compound is provided after the cell culture has begun.

In certain embodiments, the concentration of an anti-senescence compound present in the cell culture is different for varying cell types and products. In certain embodiments, the concentration of carnosine present is different for varying cell types and products. Generally, the concentration is enough to enhance the productivity and quality without toxic effects. For the instant examples, the range includes, but is not limited to, 5 mM to 100 mM. It will be appreciated that the concentration of carnosine used may vary depending on the cell culture medium. The appropriate concentration of carnosine for a particular cell line may need to be determined with routine small-scale experiments, such as, for example, a 2L bioreactor, using conventional methods. One of ordinary skill in the art will be able to determine an advantageous or optimal concentration of carnosine or other anti-senescence compound without undue experimentation using cell culture techniques and diagnostic methods that are known in the art.

One advantage to adding carnosine or another anti-senescence compound, rather than chemical agents, is the effect on viability. Typically, cell growth ceases and the viable cell number decreases with the addition of a chemical agent, like sodium butyrate. (Kim et al. Biotechnol Bioeng, 71: 184-193,184). Examples below, however, demonstrate that addition of carnosine to a cell culture medium results in higher viability at the time of harvest than is observed in a cell culture grown in the absence of an anti-senescence compound such as carnosine. Furthermore, such carnosine-containing cell cultures exhibit an increase in specific productivity. With the positive effects on the cell viability and specific productivity, the overall yield is higher.

Another advantage is that an anti-senescence compound such as carnosine decreases the amount of high molecular weight aggregates and/or the number of acidic species. Decreasing the amount of high molecular weight aggregates and acidic species simplifies purification of the protein product. Enabling the protein to be isolated more efficiently decreases the cost to produce the protein product. In certain embodiments, an anti-senescence compound other than carnosine is used to decrease the amount of high molecular weight aggregates and/or acidic species. In certain embodiments, two or more anti-senescence compounds are used to decrease the amount of high molecular weight aggregates and/or acidic species.

In certain embodiments, cells are grown in accordance with any of the cell culture methods described in United States Patent Application Serial Nos. 11/213,308, 11/213,317 and 11/213,633 each of which was filed August 25, 2005, and each of which is herein incorporated by reference in its entirety. For example, in certain embodiments, the cells may be grown in a culture medium in which the cumulative amino acid concentration is greater than about 70 mM. In certain embodiments, the cells may be grown in a culture medium in which the molar cumulative glutamine to cumulative asparagine ratio is less than about 2. In certain embodiments, the cells may be grown in a culture medium in which the molar cumulative glutamine to cumulative total amino acid ratio is less than about 0.2. In certain embodiments, the cells may be grown in a culture medium in which the molar cumulative inorganic ion to cumulative total amino acid ratio is between about 0.4 to 1. In certain embodiments, the cells may be grown in a culture medium in which the combined cumulative glutamine and cumulative asparagine concentration is between about 16 and 36 mM. In certain embodiments, the cells may be grown in a culture medium that contains two, three, four or all five of the preceding medium conditions. Use of such media allows high levels of protein production and lessens accumulation of certain undesirable factors such as ammonium and/or lactate.

In some embodiments, the cells are grown under one or more of the conditions described in United States Provisional Patent Application Serial No. 60/830,658, filed July 13, 2006 and incorporated herein by reference in its entirety. For example, in some embodiments, cells are grown in a culture medium that contains manganese at a concentration between approximately 10 and 600 nM. In some embodiments, cells are grown in a culture medium that contains manganese at a concentration between approximately 20 and 100 nM. In some embodiments, cells are grown in a culture medium that contains manganese at a concentration of approximately 40 nM. Use of such media in growing glycoproteins results in production of a glycoprotein with an improved glycosylation pattern (e.g. a greater number of covalently linked sugar residues in one or more oligosaccharide chains).

In certain embodiments of the invention, proteins produced according to one or more methods of the present invention will have pharmacologic activity and will be useful in the preparation of pharmaceuticals. Proteins produced according to one or more methods of the present invention may be administered to a subject or may first be formulated for delivery by any available route including, but not limited to parenteral (e.g., intravenous), intradermal, subcutaneous, oral, nasal, bronchial, opthalmic, transdermal (topical), transmucosal, rectal, and vaginal routes. Inventive pharmaceutical compositions typically include a purified protein expressed from a mammalian cell line, a delivery agent (i.e., a cationic polymer, peptide molecular transporter, surfactant, etc., as described above) in combination with a pharmaceutically acceptable carrier. As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Such formulations will be known by those of skill in the art. In certain embodiments, a protein produced according to the present invention is formulated in oral and/or parenteral form. In certain embodiments, for ease of administration and uniformity of dosage, such oral and/or parenteral forms are formulated as unit dosage form, wherein each unit contains a predetermined quantity of active protein calculated to produce the desired therapeutic effect in association with the required pharmaceutical carrier. One of ordinary skill in the art will be aware of unit dosage formulations appropriate for proteins produced according to the present invention.

Certain embodiments and aspects are discussed in detail above. The present disclosure is further illustrated by the following, non-limiting examples. Those of ordinary skill in the art will understand, however, that various modifications to these embodiments are within the scope of the appended claims. It is noted that the addition of carnosine and/or other anti-senescence compounds is equally applicable to other mammalian cell cultures and protein products. It is the claims and equivalents thereof that define the scope of the present invention, which is not and should not be limited to or by this description of certain embodiments.

### EXAMPLES

### Example 1

### Dish Scale Carnosine Experiments

A MYO-29 cell line was cultured in a serum free production medium in a bioreactor with a 1 L working volume and was temperature shifted from 37°C to 31°C on day 4, The pH of the bioreactor was held at 7.00 and the dissolved oxygen was at 30% air saturation. Cell culture medium was then taken from the bioreactor on day 4, day 7, and day 10 and put into culture dishes with an 8 ml working volume and placed into a 31°C incubator, where the dish cultures were cultured until day 12. The cells were supplemented with a feed medium on days 5 and 7. On day 5, 10 %(v/v) of feed medium was added to the cell cultures and on day 7, 5%(v/v) of feed medium was added to the cell cultures. 10 mM of carnosine was added on day 4, day 7, and day 10, respectively, to the dishes and the cell culture medium was harvested on day 12.

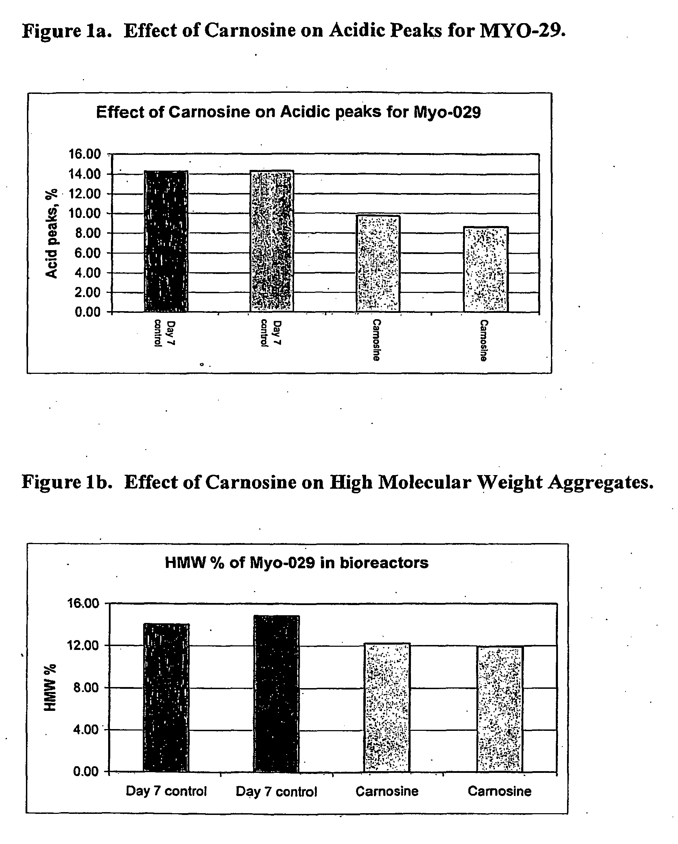

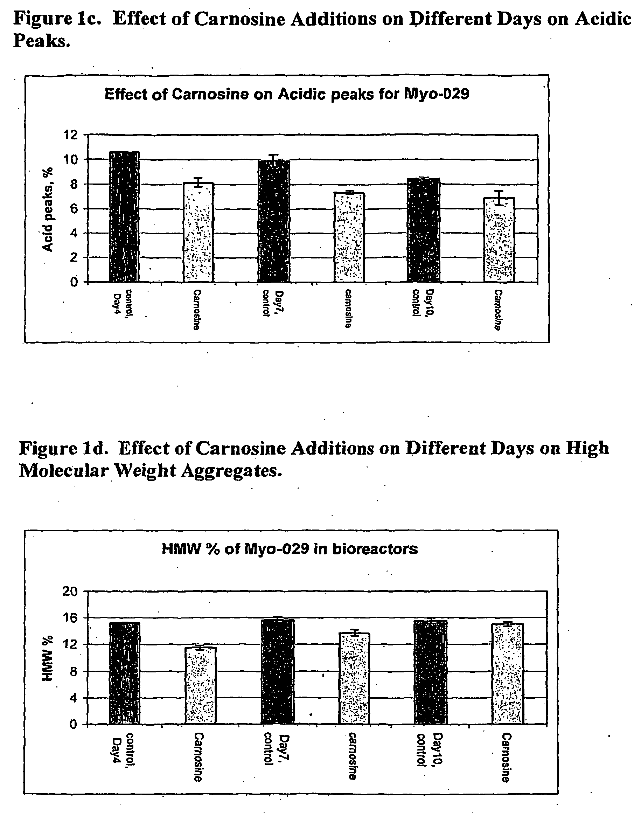

Figure 1a shows the effect of the carnosine additions on the amount of acidic peaks in the culture on day 7. Figure 1b shows the effect of the carnosine on the high molecular weight aggregates on the day 7 culture. Figure 1c illustrates the effect of carnosine additions on day 4, versus day 7, versus day 10 on the acidic peaks. Figure 1d shows the same experiment's results but for high molecular weight aggregates. Overall, carnosine had a positive effect by decreasing both the acidic peaks and the high molecular weight aggregates in cultured dishes.

### Example 2

### Effects of Carnosine Addition to Cell Culture Medium

Five bioreactors were inoculated with 0.9 x10⁶ cells/ml with a 1L working volume of a MYO-29 cell line in a serum free inoculation medium. All the bioreactors were fed 5% (v/v) of a feed medium on days 3, 5, 7, and 12 of a 14-day run. A day 10 feed of 5% (v/v) of the feed medium was added to two of the control bioreactors and one containing carnosine. The conditions of the bioreactors were held at a temperature of 37°C, a pH of 7.00, and a dissolved oxygen level at 30% air saturation. The agitation rate was 200 rpm and the sparge gas had a combination of air and 7% carbon dioxide.

All cells were cultured for 4 days at which point 20 mM of carnosine was added to two of the bioreactors, the controls did not have carnosine added, and the temperature was shifted to 31°C in all of the bioreactors on day 4 also. The bioreactors were harvested on day 14 of the production run. Samples were taken throughout the run to monitor the progress of the cell culture medium. The controls performed as expected.

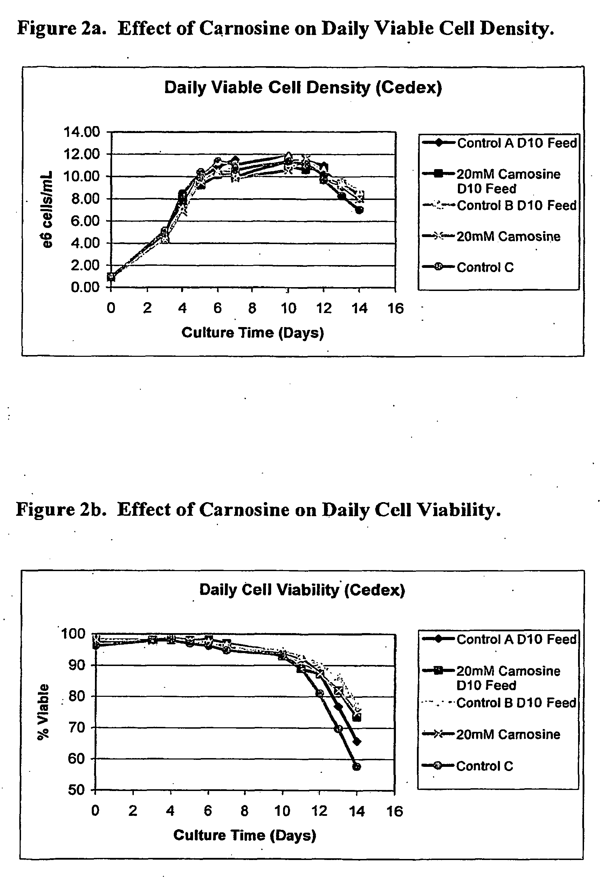

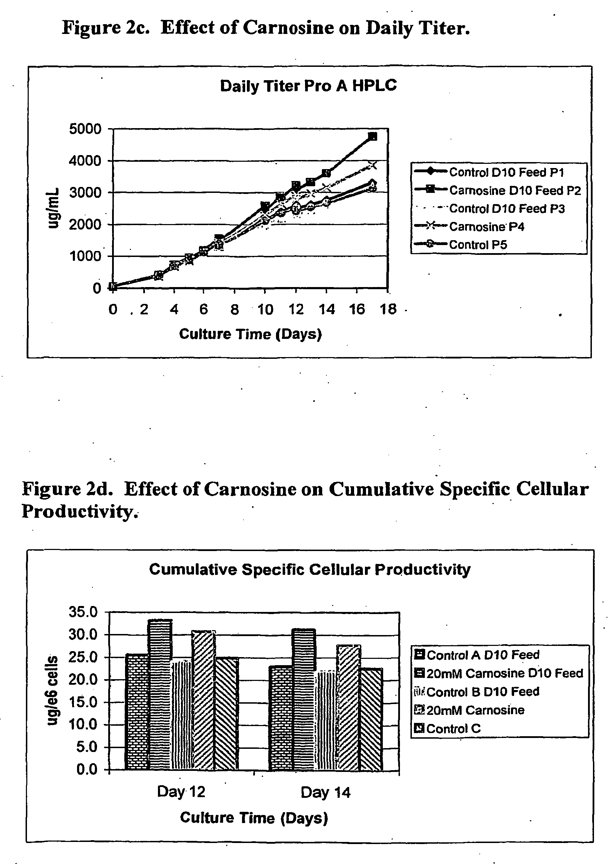

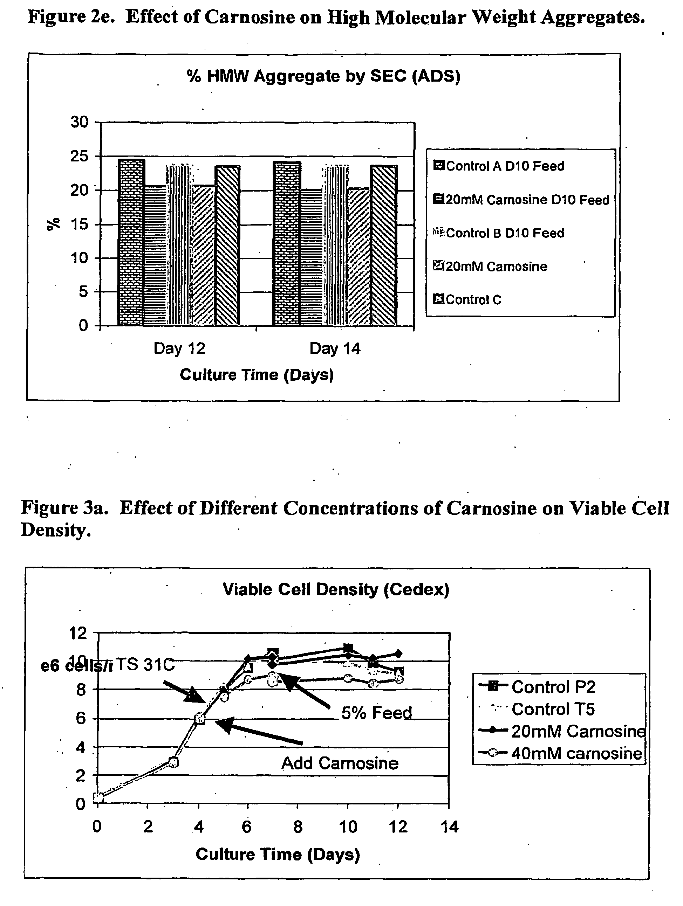

Figure 2a shows the daily viable cell density. Figure 2b shows that the daily cell viability of the bioreactors with carnosine was higher upon being harvested compared to two bioreactors without it. Figure 2c shows the daily titer of the bioreactors and that the two bioreactors with carnosine present had higher titer at the time of harvest. The cultures with carnosine had better cumulative specific cellular productivity shown in Figure 2d. Figure 2e shows the amount of high molecular weight aggregates and shows a decrease of high molecular weight aggregates in the bioreactors with carnosine present.

### Example 3

### Effect of Different Concentration of Carnosine Additions

Four bioreactors were inoculated with 0.4 x 10⁶ cells/ml with a 1L working volume of a MYO-29 cell line in a serum free inoculation medium. All the bioreactors were all fed 5% (v/v) of a feed medium on day 7 of the 14-day run. The conditions of the bioreactor were held at a temperature of 37°C, a pH of 7.00, and a dissolved oxygen level at 30% air saturation. The agitation rate was 200 rpm and the sparge gas had a combination of air and 7% carbon dioxide.

All cells were cultured for four days at which point the temperature was shifted in all of the bioreactors to 31°C. Also on day 4, 20 mM of carnosine was added to one bioreactor, a second had 40 mM of carnosine added, and the controls did not have any carnosine added. All bioreactors were harvested on day 12 of the production run. Samples were taken throughout the run to monitor the progress of the cell culture medium. The controls performed generally as expected, except one control had slightly lower daily viabilities than previously seen.

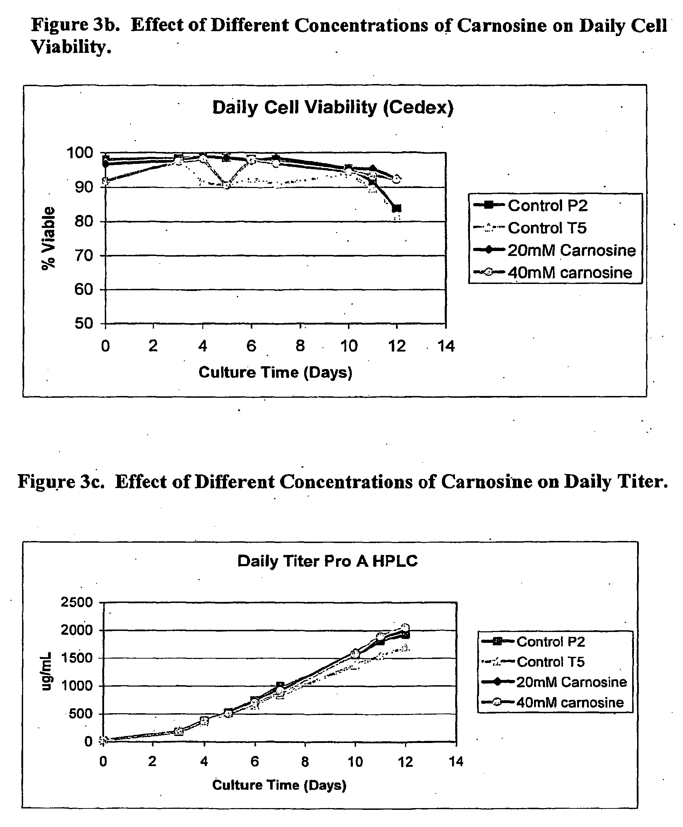

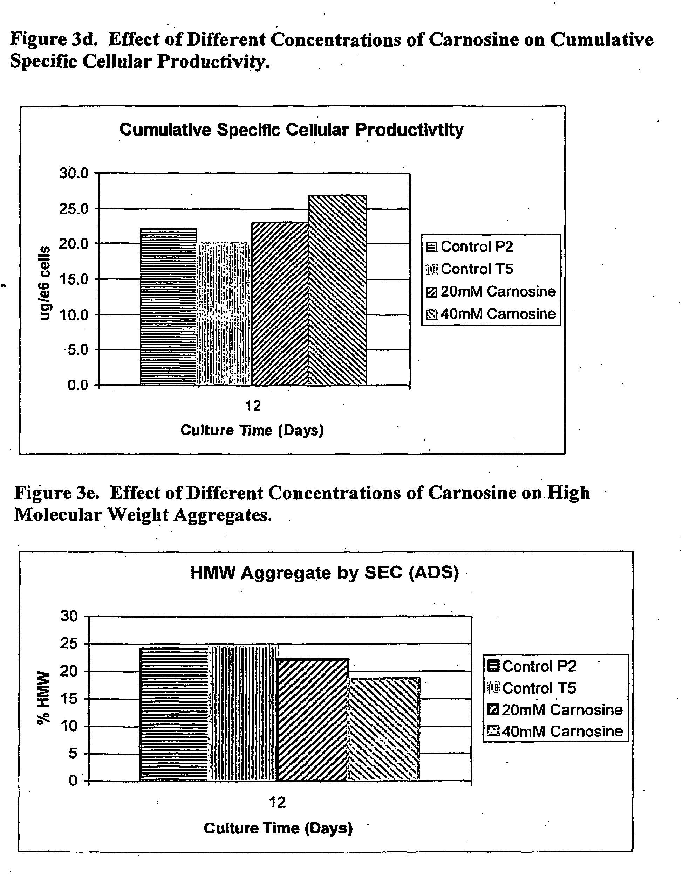

Figure 3a shows the daily viable cell density, the different bioreactors are fairly similar with the exception of the bioreactor with 40 mM of carnosine. Figure 3b shows that the daily cell viability of the bioreactors with carnosine had a higher viability upon being harvested compared to two control bioreactors without it. Figure 3c shows the daily titer; the bioreactors with the carnosine additions and one of the controls were similar. The bioreactor with 40 mM of carnosine had a higher cumulative specific cellular productivity. (Figure 3d). Figure 3e shows the amount of high molecular weight aggregates; overall there is a decrease in high molecular weight aggregates with the carnosine additions compared to the controls.

## Claims

1. A method of producing a protein in cell culture comprising steps of:
culturing mammalian cells transformed with a gene encoding a protein of interest, which gene is expressed under conditions of cell culture, in a cell culture medium comprising an anti-senescence compound selected from carnosine, acetyl-carnosine, homo-carnosine, anserine, and β-alanine; wherein the anti-senescence compound is at a concentration between about 5 mM to about 100 mM, and
maintaining the culture under conditions and for a time sufficient to permit expression of the protein;
wherein the cell culture exhibits an improved cell culture characteristic that differs from a corresponding cell culture characteristic that would be observed under otherwise identical conditions in an otherwise identical medium that lacks the antisenescence compound;
wherein the improved culture characteristic is selected from the group consisting of: increased titer, increased cell specific productivity, decreased accumulation of high molecular weight aggregates and decreased accumulation of acidic species, and combinations thereof.

2. The method of claim 1 wherein said anti-senescence compound is carnosine.

3. The method of claim 1, wherein said mammalian cell is a CHO cell.

4. The method of claim 1, wherein said protein of interest is an antibody.

5. The method of claim 4, wherein said antibody is an IgG isotope.

6. The method of claim 4, wherein said antibody is a recombinant human antibody.

7. The method of claim 4, wherein said antibody is specifically reactive with growth differentiation factor-8 (GDF-8) or an epitope thereof.

8. The method of claim 4, wherein said recombinant antibody specifically binds to BMP-11.

9. The method of claim 1, wherein said anti-senescence compound is at a concentration between about 10 mM to about 40 mM.

10. The method of claim 1, wherein said anti-senescence compound is at a concentration of about 20 mM.

11. The method of claim 1, wherein said anti-senescence compound is added in combination with a feed medium.

12. The method of claim 1, wherein said cell viability at time of harvest is maintained.

13. The method of claim 1, wherein production of said protein of interest is increased.

14. The method of claim 1, wherein cell specific productivity of said protein of interest is increased.

15. The method of claim 1, wherein quality of said protein of interest is increased by a decrease in high molecular weight aggregate.

16. The method of claim 1, wherein quality of said protein of interest is increased by a decrease in acidic species.

## Patentansprüche

1. Verfahren zur Herstellung eines Proteins in Zellkultur, umfassend die Schritte:
Kultivieren von Säugerzellen, die mit einem Gen transformiert wurden, das ein Protein von Interesse codiert, wobei das Gen unter Zellkulturbedingungen in einem Zellkulturmedium, das eine Anti-Seneszenz-Verbindung, ausgewählt aus Carnosin, Acetyl-Carnosin, Homocarnosin, Anserin und β-Alanin, umfasst, exprimiert wird; wobei die Anti-Seneszenz-Verbindung mit einer Konzentration zwischen etwa 5 mM und etwa 100 mM vorliegt, und
Aufrechterhalten der Kultur unter Bedingungen und für eine Zeit, die ausreichend sind, um eine Expression des Proteins zu erlauben;
wobei die Zellkultur ein verbessertes Zellkulturcharakteristikum aufweist, das sich von einem entsprechenden Zellkulturcharakteristikum unterscheidet, das unter ansonsten identischen Bedingungen in einem ansonsten identischen Medium, dem die Anti-Seneszenz-Verbindung fehlt, beobachtet würde;
wobei das verbesserte Kulturcharakteristikum ausgewählt ist aus der Gruppe, bestehend aus: erhöhtem Titer, erhöhter zellspezifischer Produktivität, verringerter Akkumulation von Aggregaten mit hohem Molekulargewicht und verringerter Akkumulation saurer Spezies und Kombinationen davon.

2. Verfahren nach Anspruch 1, wobei die Anti-Seneszenz-Verbindung Carnosin ist.

3. Verfahren nach Anspruch 1, wobei die Säugerzelle eine CHO-Zelle ist.

4. Verfahren nach Anspruch 1, wobei das Protein von Interesse ein Antikörper ist.

5. Verfahren nach Anspruch 4, wobei der Antikörper ein IgG-Isotop ist.

6. Verfahren nach Anspruch 4, wobei der Antikörper ein rekombinanter humaner Antikörper ist.

7. Verfahren nach Anspruch 4, wobei der Antikörper mit Wachstumsdifferenzierungsfaktor-8 (GDF-8) oder einem Epitop davon spezifisch reaktiv ist.

8. Verfahren nach Anspruch 4, wobei der rekombinante Antikörper spezifisch an BMP-11 bindet.

9. Verfahren nach Anspruch 1, wobei die Anti-Seneszenz-Verbindung mit einer Konzentration zwischen etwa 10 mM und 40 mM vorliegt.

10. Verfahren nach Anspruch 1, wobei die Anti-Seneszenz-Verbindung mit einer Konzentration von etwa 20 mM vorliegt.

11. Verfahren nach Anspruch 1, wobei die Anti-Seneszenz-Verbindung in Kombination mit einem Fütterungsmedium zugesetzt wird.

12. Verfahren nach Anspruch 1, wobei die Zelllebensfähigkeit zur Zeit der Ernte aufrecht erhalten wird.

13. Verfahren nach Anspruch 1, wobei die Produktion des Proteins von Interesse erhöht wird.

14. Verfahren nach Anspruch 1, wobei die zellspezifische Produktivität des Proteins von Interesse erhöht wird.

15. Verfahren nach Anspruch 1, wobei die Qualität des Proteins von Interesse durch eine Verringerung beim Aggregat mit hohem Molekulargewicht erhöht wird.

16. Verfahren nach Anspruch 1, wobei die Qualität des Proteins von Interesse durch eine Verringerung der sauren Spezies erhöht wird.

## Revendications

1. Procédé de production d'une protéine en culture cellulaire comprenant les étapes suivantes :
la culture de cellules de mammifères transformées avec un gène codant pour une protéine d'intérêt, lequel gène est exprimé dans des conditions de culture cellulaire, dans un milieu de culture cellulaire comprenant un composé anti-sénescence choisi parmi la carnosine, l'acétyl-carnosine, l'homocarnosine, l'ansérine, et la β-alanine ; le composé anti-sénescence étant à une concentration entre environ 5 mM et environ 100 mM, et
le maintien de la culture dans des conditions et pendant un temps suffisant pour permettre l'expression de la protéine ;
la culture cellulaire présentant une amélioration de la caractéristique de culture cellulaire qui diffère de la caractéristique d'une culture cellulaire correspondante qui serait observée dans des conditions autrement identiques dans un milieu autrement identique auquel il manque le composé anti-sénescence ;
l'amélioration de la caractéristique de culture étant choisie dans le groupe constitué de : une augmentation du titre, une augmentation de la productivité spécifique de la cellule, une diminution de l'accumulation d'agrégats de masse moléculaire élevée et une diminution de l'accumulation des espèces acides, et des combinaisons de celles-ci.

2. Procédé selon la revendication 1, dans lequel l'agent anti-sénescence est la carnosine.

3. Procédé selon la revendication 1, dans lequel ladite cellule de mammifère est une cellule CHO.

4. Procédé selon la revendication 1, dans lequel ladite protéine d'intérêt est un anticorps.

5. Procédé selon la revendication 4, dans lequel ledit anticorps est un isotope IgG.

6. Procédé selon la revendication 4, dans lequel ledit anticorps est un anticorps humain recombinant.

7. Procédé selon la revendication 4, dans lequel ledit anticorps est spécifiquement réactif avec le facteur de différenciation de la croissance 8 (GDF-8) ou un épitope de celui-ci.

8. Procédé selon la revendication 4, dans lequel ledit anticorps recombinant se lie spécifiquement à la BMP-11.

9. Procédé selon la revendication 1, dans lequel ledit composé anti-sénescence est à une concentration entre environ 10 mM et environ 40 mM.

10. Procédé selon la revendication 1, dans lequel ledit composé anti-sénescence est à une concentration d'environ 20 mM.

11. Procédé selon la revendication 1, dans lequel ledit composé anti-sénescence est ajouté en combinaison avec un milieu de charge.

12. Procédé selon la revendication 1, dans lequel ladite viabilité cellulaire au moment de la récolte est maintenue.

13. Procédé selon la revendication 1, dans lequel la production de ladite protéine d'intérêt est augmentée.

14. Procédé selon la revendication 1, dans lequel la productivité spécifique de la cellule de ladite protéine d'intérêt est augmentée.

15. Procédé selon la revendication 1, dans lequel la qualité de ladite protéine d'intérêt est augmentée par une diminution des agrégats de masse moléculaire élevée.

16. Procédé selon la revendication 1, dans lequel la qualité de ladite protéine d'intérêt est augmentée par une diminution des espèces acides.
